# EUROPEAN PATENT APPLICATION

(11) **EP 3 790 018 A1**
(43) Date of publication of application: **10.03.2021**
(21) Application number: 19853138.6
(22) Date of filing: 07.08.2019
(51) Int. Cl.: G16H 20/10, G16H 70/40, G16H 80/00, G06Q 30/02, G16H 50/20

(54) **CONTROL METHOD FOR ELECTRONIC DEVICE, AND COMPUTER-READABLE RECORDING MEDIUM FOR STORING PROGRAM FOR PERFORMING SAME**

(30) Priority: 21.08.2018 KR 20180097593
(71) Applicant: Samsung Electronics Co., Ltd., Suwon-si 16677 (KR)
(72) Inventor: OH, Youngjae, Suwon-si Gyeonggi-do 16677 (KR); KIM, Kwangbok, Suwon-si Gyeonggi-do 16677 (KR); CHO, Chulho, Suwon-si Gyeonggi-do 16677 (KR); KWON, Dongwook, Suwon-si Gyeonggi-do 16677 (KR); CHO, Seongje, Suwon-si Gyeonggi-do 16677 (KR)
(74) Representative: Nederlandsch Octrooibureau
(86) International application number: PCT/KR2019/009875
(87) International publication number: WO 2020/040455

(57) **Abstract**

A control method for an electronic apparatus, and a computer-readable recording medium for storing a program for performing same are provided. The control method for an electronic apparatus can transmit a payment request for medicine to an external device, receive medication information for the medicine from the external device in response to the payment request, store the medication information for the medicine, and provide a medication alarm for the medicine to a user on the basis of the stored medication information.

## Description

### [Technical Field]

The disclosure relates to a method of an electronic apparatus and a computer-readable recording medium storing a program for performing the same. More particularly, the disclosure relates to a method of controlling an electronic apparatus that receives medication information from an external device and provides a medication alarm to a user, and a computer-readable recording medium storing a program for performing the same.

### [Background Art]

As the aging population increases, the number of patients with chronic diseases is increasing. Since patients with chronic diseases have to take medicine at an appointed time, it is very important for patients with chronic diseases to manage medication.

Conventionally, a pharmacist guided the user about the type of medicine to be taken orally, when to take the medicine, and how to take the medicine. However, in this case, many of medicine takers are taking medicine incorrectly since they take the medicine depending on their memory. Accordingly, a duration of taking medicine is increased, which has an adverse effect on health and increases the cost of medicine.

Conventionally, users used to input medication information into an electronic apparatus such as smartphones or photograph a barcode of a prescription to acquire medication information. In this case, the users had to directly input medication information or photograph prescriptions.

Accordingly, there is a growing need for more simply acquiring medication information on an electronic apparatus and providing a medication alarm to the user according to the acquired medication information.

### [Detailed Description of the Invention]

### [Technical Problem]

The disclosure is to solve the problem described above, and the disclosure receives medication information at the same time as payment, and provides a medication alarm based on the received medication information, thereby providing a method of controlling an electronic apparatus capable of providing more convenient and accurate medication management service to the user, and a computer-readable recording medium storing a program for performing the same.

### [Technical Solution]

According to an embodiment of the disclosure, a method of controlling an electronic apparatus includes transmitting a payment request for medicine to an external device, receive medication information for the medicine from the external device in response to the payment request, storing the medication information for the medicine, and providing a medication alarm for the medicine to a user based on the stored medication information.

According to an embodiment of the disclosure, a computer-readable recording medium for storing computer executable instructions for performing a method of controlling an electronic apparatus, the medium includes transmitting a payment request for medicine to an external device, storing the medication information for the medicine based on the medication information for the medicine being received from the external device in response to the payment request, and providing a medication alarm for the medicine to a user based on the stored medication information.

### [Effect of the Invention]

According to an embodiment of the disclosure as described above, the electronic apparatus can acquire medication information without a separate user operation and provide a medication alarm based on the medication information. Accordingly, the user can be provided with more convenient and accurate medication management service.

### [Brief Description of Drawings]

FIG. 1 is a conceptual diagram illustrating a method of providing a medication alarm service according to an embodiment of the disclosure;
FIG. 2 is a view illustrating a system for providing a medication alarm service according to an embodiment of the disclosure;
FIG. 3 is a block diagram illustrating a configuration of an electronic apparatus according to an embodiment of the disclosure;
FIGS. 4 to 9 are views illustrating an embodiment of acquiring medication information from a pharmacy system according to various embodiments of the disclosure;
FIG. 10 is a view illustrating an embodiment of providing a medication alarm according to an apparatus used by a user according to an embodiment of the disclosure;
FIG. 11 is a view illustrating an embodiment of providing a medication alarm according to a medication time according to an embodiment of the disclosure; and
FIG. 12 is a flowchart illustrating a method of controlling an electronic apparatus according to an embodiment of the disclosure.

### [Best Mode for Implementing the Disclosure]

The above and other aspects, features, and advantages of certain embodiments of the disclosure will be more apparent from the following description taken in conjunction with the accompanying drawings, in which: However, it should be understood that the disclosure is not limited to the specific embodiments described hereinafter, but includes various modifications, equivalents, and/or alternatives of the embodiments of the disclosure. In relation to explanation of the drawings, similar drawing reference numerals may be used for similar constituent elements.

The terms "have", "may have", "include", and "may include" used in the exemplary embodiments of the present disclosure indicate the presence of corresponding features (e.g., elements such as numerical values, functions, operations, or parts), and do not preclude the presence of additional features.

In the description, the term "A or B", "at least one of A or/and B", or "one or more of A or/and B" may include all possible combinations of the items that are enumerated together. For example, the term "A or B" or "at least one of A or/and B" may designate (1) at least one A, (2) at least one B, or (3) both at least one A and at least one B.

The expression "1", "2", "first", or "second" as used herein may modify a variety of elements, irrespective of order and/or importance thereof, and only to distinguish one element from another. Accordingly, without limiting the corresponding elements.

The expression "1", "2", "first", or "second" as used herein may modify a variety of elements, irrespective of order and/or importance thereof, and only to distinguish one element from another. Accordingly, without limiting the corresponding elements. When an element (e.g., a first element) is "operatively or communicatively coupled with/to" or "connected to" another element (e.g., a second element), an element may be directly coupled with another element or may be coupled through the other element (e.g., a third element).

In the description, the term "configured to" may be changed to, for example, "suitable for", "having the capacity to", "designed to", "adapted to", "made to", or "capable of' under certain circumstances. The term "configured to (set to)" does not necessarily mean "specifically designed to" in a hardware level. Under certain circumstances, the term "device configured to" may refer to "device capable of' doing something together with another device or components. For example, "a sub-processor configured (or configured to) perform A, B, and C" may refer to a generic-purpose processor (e.g., central processing unit (CPU) or an application processor) capable of performing corresponding operations by executing a dedicated processor (e.g., an embedded processor) or one or more software programs stored in a memory device to perform the operations.

An electronic apparatus according to various exemplary embodiments may include at least one of, for example, smartphone, tablet PC, mobile phone, video phone, e-book reader, desktop PC, laptop PC, netbook computer, workstation, server, PDA, portable multimedia player (PMP), MP3 player, medical device, camera, or a wearable device. For example, wearable devices may include at least one of accessories (e.g. watches, rings, bracelets, anklets, necklaces, glasses, contact lenses, or head-mounted-devices (HMD)), fabrics or clothing (e.g. electronic clothing), a body attachment type (e.g., a skin pad or a tattoo), or a bio-implantable circuit.

The pharmacy system according to various embodiments of the disclosure may include at least one of a desktop PC, a laptop PC, a netbook computer, a workstation, a server, various medical devices, a kiosk, a beacon server, and a beacon transmitter included in the pharmacy.

Hereinafter, the disclosure will be described in detail with reference to the drawings.

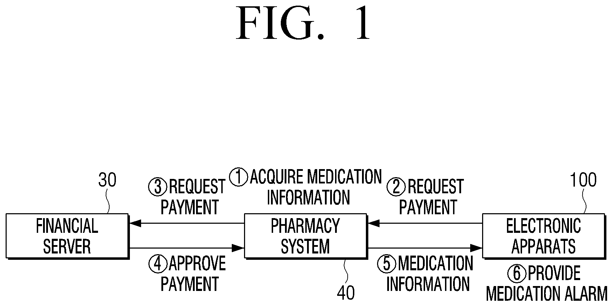

FIG. 1 is a view illustrating a method for providing a medication alarm service according to an example embodiment. First, a pharmacy system 40 may acquire medication information for a patient. At this time, the pharmacy system 40 can acquire medication information for a patient from an external hospital server, acquire medication information by photographing a barcode (or QR code, etc.) included in a prescription provided by the user, or acquire medication information directly input by a pharmacist.

The electronic apparatus 100 may request payment to the pharmacy system 40. In this case, the electronic apparatus 100 may transmit a payment token to a payment terminal connected to the pharmacy system 40 through a first communication module (e.g., a magnetic secure transmission (MST) communication module) included in the electronic apparatus 100.

The pharmacy system 40 may transmit a payment request to a financial server 30 based on the payment token acquired through the payment terminal. The financial server 30 may approve payment in response to the payment request and transmit a payment approval signal to the pharmacy system 40. In this case, the financial server 30 may transmit the payment approval signal to the electronic apparatus 100 together.

When the payment approval signal is received, the pharmacy system 40 may provide medication information to the electronic apparatus 100. In this case, the electronic apparatus 100 may receive the medication information through a second communication module (e.g., an NFC communication module).

In addition, the electronic apparatus 100 may perform a validity check on the received medication information. For example, the electronic apparatus 100 may compare user information stored in the electronic apparatus 100 with user information included in the medication information, or compare payment information received by the electronic apparatus 100 with payment information included in the medication information to perform the validity check. When the validity of medication information is authenticated, the electronic apparatus 100 may set a medication alarm based on the medication information. For example, the electronic apparatus 100 may set a medication alarm based on information on medication time, a type of medicines and a method of taking medicines included in medication information.

The electronic apparatus 100 may provide a medication alarm. Specifically, the electronic apparatus 100 may provide a medication alarm at an alarm time set based on a medication time included in the medication information. In this case, the medication alarm may include information on the type of medication and how to take medicine, and the medication alarm may be provided using at least one of a visual message, an audible message, and a tactile message.

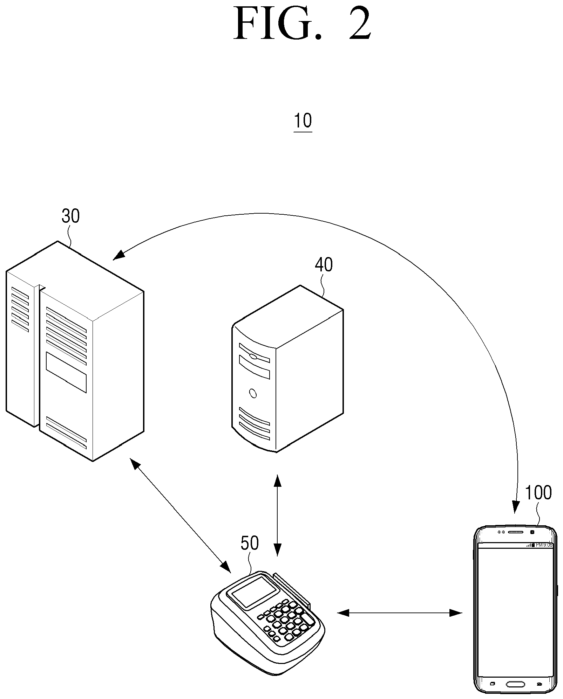

FIG. 2 is a view illustrating a system for providing a medication alarm service according to an embodiment of the disclosure. The system 10 may include a financial server 30, a pharmacy system 40, a payment terminal 50, and an electronic apparatus 100.

The financial server 30 may receive a signal acquired from the payment terminal 50. Specifically, when the electronic apparatus 100 contacts the payment terminal 50 such that the payment terminal 50 receives a payment token from the electronic apparatus 100 through a near-field communication module (e.g., an MST communication module, an NFC communication module, etc.), the financial server 30 may receive a payment request based on the payment token from the payment terminal 50.

The financial server 30 may transmit a payment approval signal to the payment terminal 50 in response to the payment request. In addition, the financial server 30 may transmit the payment approval signal to the electronic apparatus 100 as well as the payment terminal 50. In this case, the payment approval signal may include information on payment details.

The pharmacy system 40 may provide a payment service, a medication information providing service, a manufacturing service based on a pharmacy application (or software).

Specifically, the pharmacy system 40 may acquire medication information. In particular, the pharmacy system 40 may acquire patient medication information from an external hospital server. Alternatively, when identification information such as a barcode or QR code included in a prescription possessed by a patient is photographed by a pharmacist, the pharmacy system 40 may acquire the patient's medication information. Alternatively, the pharmacy system 40 may acquire medication information by a pharmacist directly inputting medication information according to the type of medicine included in the prescription. In this case, the medication information may include information on medicine that the patient takes, information on medication time, information on the number of times to take medicine, information on how to take medicine, or the like.

When the medication information is acquired, the pharmacy system 40 may enter a payment mode. During the payment mode, the pharmacy system 40 may transmit a signal for payment preparation to the connected payment terminal 50. While maintaining the payment mode, the pharmacy system 40 may wait for a medication information providing mode.

When payment is approved by the financial server 30, the pharmacy system 40 may transmit medication information to the electronic apparatus 100. In this case, the pharmacy system 40 may transmit medication information to the electronic apparatus 100 using a near-field wireless communication module. However, the pharmacy system 40 may provide the medication information to the electronic apparatus 100 using a communication module (e.g., NFC communication module) different from a communication module (e.g., MST communication module) to which the electronic apparatus 100 transmits a payment token for payment.

The payment terminal 50 may be connected to the pharmacy system 40 to perform a payment function for a patient's medicine. Specifically, the payment terminal 50 may receive the payment token from the electronic apparatus 100 of the patient with a near-field communication module (e.g., MST communication module), and transmit a payment request to the financial server 30 based on the received payment token. In addition, when a payment approval signal is received from the financial server 30, the payment terminal 50 may transmit a payment approval signal to the pharmacy system 40.

The electronic apparatus 100 may provide a medication service to the user through various applications such as a payment application, a medication application, or the like. In particular, the electronic apparatus 100 may perform payment for medicine using a payment application using a near-field communication module. For example, the electronic apparatus 100 may transmit a payment token to the payment terminal 50 using the MST communication module (or NFC communication module). In this case, the electronic apparatus 100 may drive a medication application to provide medication information, and activate the near-field wireless communication (e.g., an NFC communication module) in a standby state of receiving.

When payment is approved, the electronic apparatus 100 may receive medication information from the pharmacy system 40. In this case, the medication information may include information on medicine to be taken by a patient, information on the time of taking medicine, information on the number of times to take medicine, information on how to take medicine, or the like.

In particular, the electronic apparatus 100 may check validity on the medication information based on user information or payment information included in the medication information. Specifically, the electronic apparatus 100 may compare the user information stored in the electronic apparatus 100 with the user information included in the medication information, or compare the payment information received by the electronic apparatus 100 with the payment information included in the medication information.

When the validity on the medication information is authenticated, the electronic apparatus 100 may set a medication alarm based on the received medication information. In this case, the electronic apparatus 100 may transmit the received medication information or information on the medication alarm to an external device (e.g., an IOT device) connected to the electronic apparatus 100.

In addition, the electronic apparatus 100 may provide a set medication alarm. In particular, the electronic apparatus 100 may provide a medication alarm based on the time of taking medicine included in the medication information. In particular, the electronic apparatus 100 may provide the medication alarm by determining the time of taking medication in a different way depending on whether the time is before or after having a meal. This will be described in detail later.

According to an embodiment of the disclosure described above, the user may be provided with the medication information for a medication alarm at the same time as a payment, thereby receiving more convenient and accurate medication information providing service.

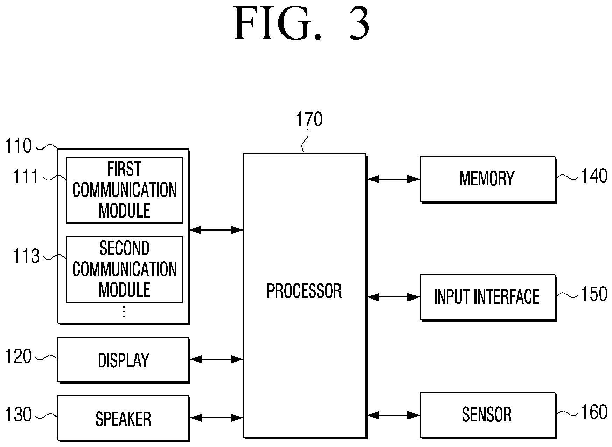

FIG. 3 is a block diagram illustrating a configuration of an electronic apparatus according to an embodiment of the disclosure. As shown in FIG. 3, the electronic apparatus 100 may include a communicator (e.g., including communication circuitry) 110, a display 120, a speaker 130, a memory 140, an input interface 150, a sensor 160, and a processor 170. Meanwhile, configurations illustrated in FIG. 3 is an exemplary view for implementing the embodiment of the disclosure shown, appropriate hardware/software configurations evident to those skilled in the art may be further included in the electronic apparatus 100, or the configuration illustrated in FIG. 3 may be omitted.

The communicator 110 may be configured to communicate with various external devices using a wireless signal. In particular, the communicator 110 may include a near-field communication module for receiving payment and medication information. In this case, the communicator 110 may include a first communication module 111 for transmitting a payment token to the payment terminal 50. The first communication module 111 may be implemented as an MST communication module. The MST communication module transmits magnetic credit card information to an external payment terminal 50 through a root antenna, and may be implemented as a separate chip. In addition, the communicator 110 may include a second communication module 113 for receiving medication information. The second communication module 113 may be implemented as an NFC communication module. In other words, the NFC communication module is a non-contact near-field wireless communication module using a 13.56MHz frequency band as one of an electronic tag (RFID), and may transmit data between terminals within a short distance of 10cm.

Meanwhile, in the embodiment described above, it has been described that the communicator 110 performs a payment function and a medication information receiving function using a plurality of near-field communication modules, but this is only an embodiment, and may provide the payment function and the medication information receiving function using one near-field communication module. For example, the payment function and the medication information receiving function may be performed using an NFC communication module capable of two-way communication. Specifically, when the electronic apparatus 100 including the NFC communication module is close to the payment terminal 50, the electronic apparatus 100 may transmit a payment token to the payment terminal 50 using the NFC communication module, and may receive medication information from the payment terminal 50 connected to the pharmacy system 40 when the payment is approved.

The communicator 110 may perform communication with various types of external devices according to various types of communication methods as well as the near-field communication module described above. The communicator 110 may include a Wireless-Fidelity (Wi-Fi) chip, a Bluetooth chip, or the like. In addition, the communicator 110 may provide a payment function and a medication information receiving function using a long-distance communication module. This will be described below with reference to the drawings.

In addition, the communicator 110 may transmit the medication information received from the pharmacy system 50 to an external device (e.g., an IOT device).

The display 120 may provide various screens. In particular, the display 120 may provide a UI screen for providing a medication alarm. In this case, the UI screen may include information on medicine taken by a patient, a method of taking medication, or the like.

The speaker 130 may output an audio signal. In particular, the speaker 130 may audibly provide information on a medication alarm. For example, when the time of taking medication is reached, the speaker 130 may provide an audible feedback (e.g., a voice message, a beep sound, etc.) indicating the medication alarm. In this case, the speaker 130 may be provided in the electronic apparatus 100, but this is only an embodiment and may be electrically connected to the electronic apparatus 100.

The memory 140 may store an instruction or data related to at least one of the other elements of the electronic apparatus 100. In particular, the memory 120 may be implemented as a non-volatile memory, a volatile memory, a flash memory, a hard disk drive (HDD), a solid state drive (SDD), or the like. The memory 120 may be accessed by the processor 170, and perform readout, recording, correction, deletion, update, and the like. According to an embodiment of the disclosure, the term of the memory may include the memory 140, read-only memory (ROM) (not illustrated) and random access memory (RAM) (not illustrated) within the processor 170, and a memory card (not illustrated) attached to the electronic apparatus 100 (e.g., micro secure digital (SD) card or memory stick).

In particular, the memory 140 may store a payment application and a medication application. In this case, the payment application is an application for performing a payment function, and may transmit a payment token to an external payment terminal through a near-field communication module. In addition, the medication application may provide a medication alarm based on medication information received from the pharmacy system 40 through the communicator 110.

The input interface 150 may receive various user inputs and transmit them to the processor 170. In particular, the input interface 150 may include a touch sensor, a (digital) pen sensor, a pressure sensor, a key, or a microphone. The touch sensor may, for example, use at least one of electrostatic type, pressure sensitive type, infrared type, and an ultraviolet type. The digital pen sensor may, for example, be part of a touch panel or include an additional sheet for recognizing use. The key, for example, may include a physical button, an optical key, or keypad. In particular, the input interface 150 may acquire an input signal according to a user input for executing an artificial intelligence-only program.

The sensor 160 may detect various environmental information. Specifically, the sensor 160 may include various sensors (e.g., a blood sugar enzyme sensor, a non-invasive blood sugar sensor, a calorie sensor, a blood pressure sensor, etc.) for acquiring the user's biometric information. In addition, the sensor 160 may include an image sensor (i.e., a camera) for photographing the surroundings of the electronic apparatus 100.

Meanwhile, the sensor 160 may be included in the electronic apparatus 100, but this is only an embodiment, and may be connected to the electronic apparatus 100 through a communication interface (e.g., Bluetooth).

The processor 170 is electrically connected to the communicator 110, the display 120, the speaker 130, the memory 140, the input interface 150, and the sensor 160 to control overall operation and function of the electronic apparatus 100. In particular, when payment is approved through the first communication module 111, the processor 170 may receive medication information on medicine through the second communication module 113 from an external device (e.g., the pharmacy system 40 or the payment terminal 50). In addition, the processor 170 may store the medication information on the medication in the memory 140 and provide a medication alarm to the user based on the stored medication information.

In particular, the processor 170 may compare the user information stored in the electronic apparatus 100 with the user information included in the medication information, or compare the payment information received by the electronic apparatus 100 with the payment information included in the medication information to check validity of the medication information. When the validity of the medication information is authenticated, the processor 170 may set a medication alarm for medicine based on the medication information, and may output a message indicating authentication failure if the validity of the medication information is not authenticated.

In addition, the processor 170 may identify an alarm time based on the time of taking medication for medicine included in the medication information, and provide a medication alarm at the identified alarm time. In particular, when the time of taking medication for medicine is after a meal, the processor 170 may identify whether the alarm time is reached. When the alarm time is reached, the processor 170 may identify whether the user had a meal, and when it is identified that the user had a meal, the processor 170 may provide a medication alarm after a predetermined time. In addition, when it is identified that the user did not have a meal, the processor 170 may provide a message for recommending meals and recommending medication to the user. In this case, the processor 170 may identify whether the user had a meal based on at least one of image information photographed by the electronic apparatus 100 or an IoT device connected to the electronic apparatus 100, the user's biometric information acquired by the sensor 160 included in the electronic apparatus 100, and card payment information received by the electronic apparatus 100. Meanwhile, when the time of taking medication for medicine is before having a meal, the processor 170 may provide the medication alarm at a default time of taking medication included in the alarm time. In this case, the default medication time may be pre-stored when the medication application is installed, and may be changed by user settings. Further, the processor 170 may collect the user's meal time of the electronic apparatus 100 and provide a medication alarm at the time updated based on the collected meal time.

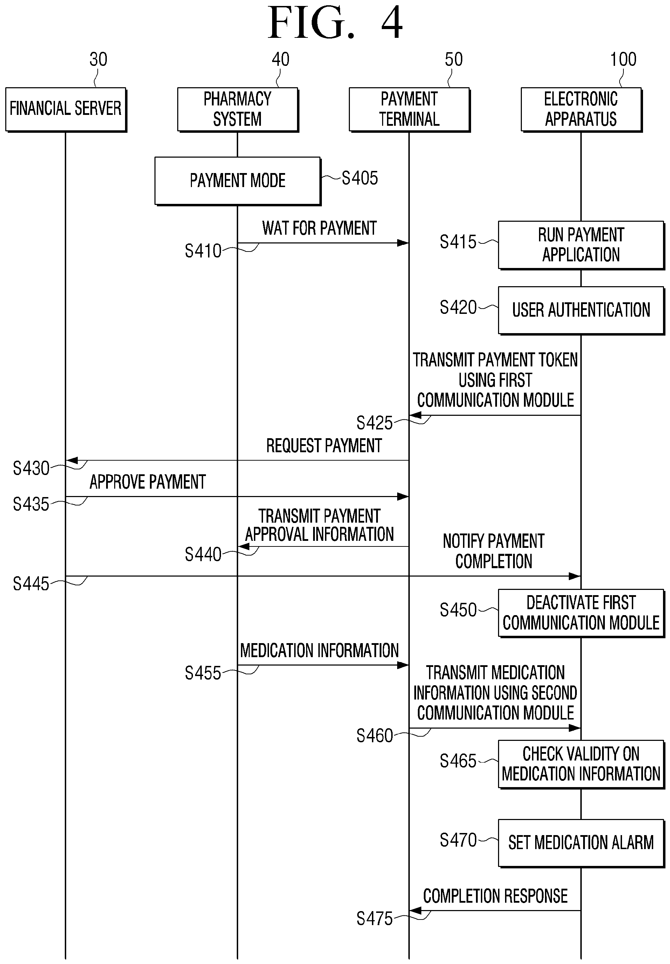

Hereinafter, various embodiments of acquiring information from a pharmacy system will be described with reference to FIGS. 4 to 9.

FIG. 4 is a view illustrating an embodiment of acquiring medication information using two communication modules according to an embodiment of the disclosure.

The pharmacy system 40 may enter a payment mode (S405). Specifically, when acquiring medication information of a patient from an external server or acquiring medication information from a prescription, the pharmacy system 40 may enter a payment mode for paying for medicine

While maintaining the payment mode, the pharmacy system 40 may request the payment terminal 50 to wait for payment (S410). In this case, the pharmacy system 40 may wait to transmit the medication information.

The electronic apparatus 100 may drive a payment application according to a user input (S415). In this case, the payment application is an application capable of performing payment using a first communication module (e.g., MST communication module) 111. In this case, while the payment application is running, the electronic apparatus 100 may also run the medication application. In this case, the medication application may be executed on a background, and when the medication application is executed, the electronic apparatus 100 may activate the second communication module (e.g., NFC communication module) 113 together. Meanwhile, as described above, it is only an embodiment that the medication application is run while the payment process is in progress, and the medication application may be run after payment for medicine is approved. Alternatively, the electronic apparatus 100 may identify a location of the electronic apparatus 100 using GPS information, network connection information, etc. included in the electronic apparatus 100, and when it is identified that the electronic apparatus 100 is in a hospital or pharmacy, the electronic apparatus 100 may run the medication application. In this case, when the medication application is run, the electronic apparatus 100 may run the payment application together. In other words, the medication application may operate in association with the payment application.

The electronic apparatus 100 may perform user authentication for payment (S420). In this case, the user authentication may be performed using user biometric information (e.g., iris, fingerprint, face, etc.) or based on a password input by the user.

When the electronic apparatus 100 approaches the payment terminal 50 after the user authentication is performed, the electronic apparatus 100 may transmit a payment token using the first communication module 111 (S425). In this case, the payment token may include information on credit card information arbitrarily allocated for security.

When the payment token is received, the payment terminal 50 may request payment to the financial server 30 (S430).

When the payment is approved based on the information included in the payment token, the financial server 30 may transmit a payment approval signal to the payment terminal 50 (S435). In this case, the payment approval signal may include information on payment details. The payment terminal 50 may transmit the payment approval information to the pharmacy system 40 (S440). Meanwhile, when the payment approval information is transmitted to the payment terminal 50 or the pharmacy system 40, the payment terminal 50 or the pharmacy system 40 may output a message to continue contacting the electronic apparatus 100 with the payment terminal 50. In this case, the message may be output through a display included in the payment terminal 50, but this is only an embodiment, and may be output through a speaker included in the payment terminal 50 or the pharmacy system 40.

In addition, the financial server 30 may transmit a signal for notification that the payment is completed to the electronic apparatus 100 (S445). In this case, information on payment details may also be included in the signal for notification of payment completion. When the signal for notification of payment completion is transmitted, the electronic apparatus 100 may deactivate the first communication module 111 for payment (S450).

The pharmacy system 40 may transmit medication information to the payment terminal 50 (S455). In this case, the pharmacy system 40 may transmit the medication information to the payment terminal 50 after payment is completed, but this is only an embodiment, and may transmit the medication information to the payment terminal 50 before the payment is completed.

The payment terminal 50 may transmit the medication information to the electronic apparatus 100 using the second communication module 113 (S460). In this case, the medication information may include information on a patient, information on payment details, information on medicines, information on the time of taking medication, information on the number of taking medication, and information on how to take medication. Meanwhile, when the electronic apparatus 100 is not close to the payment terminal 50 to transmit the medication information through the second communication module 113, the payment terminal 50 may output a message requesting approach of the electronic apparatus 100.

Meanwhile, in the embodiment described above, it has been described that the pharmacy system 40 transmits medication information to the electronic apparatus 100 through the payment terminal 50, but this is only an embodiment, and the pharmacy system 40 may directly transmit the medication information to the electronic apparatus 100.

The electronic apparatus 100 may check a validity on the received medication information (S465). Specifically, the electronic apparatus 100 may compare information on a patient included in the medication information (e.g., patient name, etc.) with information on a user stored in the electronic apparatus 100 (e.g., the name of users who logged in the medication application) to check validity on the medication information. Alternatively, the electronic apparatus 100 may compare the payment details included in the medication information with the payment details included in the notification of payment completion transmitted from the financial server 30 to the electronic device 100 to check the validity on the medication information.

When the validity of the medication information is authenticated, the electronic apparatus 100 may set a medication alarm for the medication based on the medication information (S470). Specifically, the electronic apparatus 100 may set a time to take medication based on information on the time of taking the medication included in the medication information and set the medication alarm to provide the medication alarm at the set time. However, if the validity of the medication information is not authenticated, the electronic apparatus 100 may output a message indicating the authentication is failed. In other words, the electronic apparatus 100 may request retransmission of the medication information or output a message that the authentication of the medication information is failed.

When the medication alarm is set, the electronic apparatus 100 may transmit a completion response to the payment terminal 50 (or the pharmacy system 40) (S475). At this time, the electronic apparatus 100 may output a message that guides the medication information is acquired through a speaker or display.

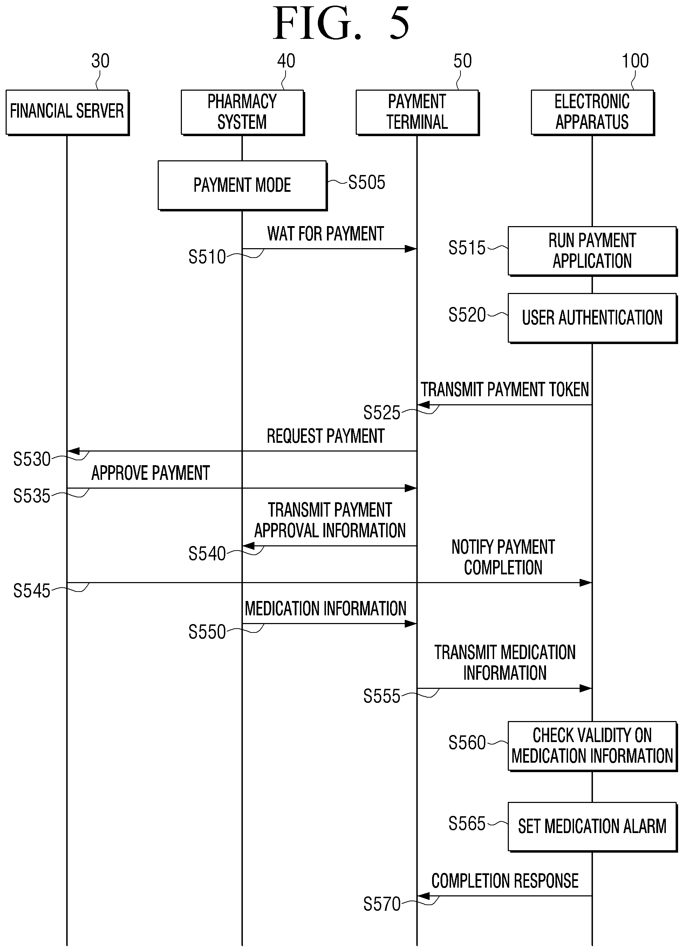

FIG. 5 is a view illustrating an embodiment of acquiring medication information using one communication module according to an embodiment of the disclosure. Operations S505 to S520 are the same as those of operations S405 to S420, and thus their repeated descriptions are omitted.

When the electronic apparatus 100 approaches the payment terminal 50 after user authentication is performed, the electronic apparatus 100 may transmit a payment token (S525). In this case, the electronic apparatus 100 may transmit the payment token using a communication module capable of bidirectional communication, such as an NFC communication module.

When the payment token is received, the payment terminal 50 may request payment to the financial server 30 (S530).

If payment is approved based on information included in the payment token, the financial server 30 may transmit a payment approval signal to the payment terminal 50 (S535). In this case, the payment approval signal may include information on payment details. Then, the payment terminal 50 may transmit payment approval information to the pharmacy system 40 (S540).

In addition, the financial server 30 may transmit a signal for notification of payment completion to the electronic apparatus 100 (S545). In this case, information on payment details may also be included in the signal for notification of payment completion. Meanwhile, in the embodiment described above, it has been described that the signal for notification of payment completion from the financial server 30 is directly transmitted to the electronic apparatus 100, but this is only an embodiment, and the financial server 30 may transmit the signal for notification of payment completion to the electronic apparatus 100.

The pharmacy system 40 may transmit medication information to the payment terminal 50 (S550). At this time, the pharmacy system 40 may transmit the medication information to the payment terminal 50 after payment is completed, but this is only an embodiment, and may transmit the medication information to the payment terminal 50 before payment is completed.

The payment terminal 50 may transmit the medication information to the electronic apparatus 100 (S555). In this case, the payment terminal 50 may transmit the medication information using a communication module (e.g., an NFC communication module) that previously transmitted the payment token.

The electronic apparatus 100 may check validity on the received medication information (S560). When the validity on the medication information is authenticated, the electronic apparatus 100 may set a medication alarm for medicine based on the medication information (S565). However, if the validity on the medication information is not authenticated, the electronic apparatus 100 may output a message indicating the authentication is failed. In other words, the electronic apparatus 100 may request retransmission of the medication information or output the message indicating that the authentication of the medication information is failed

When the medication alarm is set, the electronic apparatus 100 may transmit a complete response to the payment terminal 50 (or the pharmacy system 40) (S570).

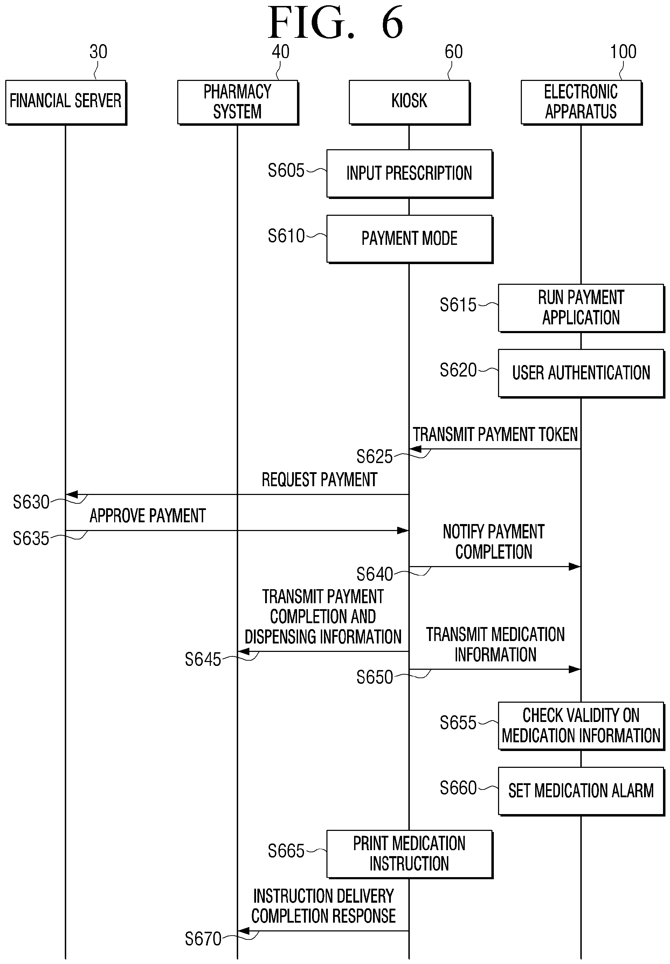

FIG. 6 is a view illustrating an embodiment that provides medication information using a kiosk according to an embodiment of the disclosure.

The kiosk 60 may receive a prescription (S605). In this case, the kiosk 60 may receive a prescription by photographing identification information such as a QR code, barcode, or the like included in the prescription. In another embodiment, the kiosk 60 may receive a prescription from an external server (e.g., a server existing in a hospital) based on patient information input by a patient.

The kiosk 60 may enter a payment mode (S610). During the payment mode, the kiosk 60 may generate medication information based on information on medicine included in the input prescription. In addition, the kiosk 60 may generate payment details based on information for the medicine included in the prescription, and wait for payment.

Meanwhile, the electronic apparatus 100 may run a payment application (S615). In this case, the electronic apparatus 100 may run the payment application based on a user input, but this is only an embodiment, and may automatically run the payment application based on an application currently executed on the electronic apparatus 100 (e.g., medication application) or a location of the electronic apparatus 100.

The electronic apparatus 100 may perform user authentication for payment (S620).

When the electronic apparatus 100 is close to an area where a communication module included in the kiosk 60 is located, the electronic apparatus 100 may transmit a payment token (S625). In this case, the payment token may be transmitted through a near-field communication module such as an NFC communication module.

When the payment token is received, the kiosk 60 may request payment to the financial server 30 (S630).

When payment is approved based on the information included in the payment token, the financial server 30 may transmit a payment approval signal to the kiosk 60 (S635). In this case, the payment approval signal may include information on payment details. In addition, the kiosk 60 may transmit a signal for notification of payment completion to the electronic apparatus 100 (S640).

The kiosk 60 may transmit a payment completion signal and dispensing information to the pharmacy system 40 (S645). In this case, the pharmacy system 40 may automatically dispense medicine based on the dispensing information or may display the dispensing information on the display in order for a pharmacist to dispense the medicine.

In addition, the kiosk 60 may transmit the acquired medication information to the electronic apparatus 100 (S650). In this case, the medication information may include information on a patient, information on payment details, information on medicine, information on the time of medication, information on the number of medications, and information on a medication method.

The electronic apparatus 100 may check validity on the received medication information (S655).

When the validity of the medication information is authenticated, the electronic apparatus 100 may set a medication alarm for medication based on the medication information (S660). Specifically, the electronic apparatus 100 may set a medication time based on information on a medication time included in the medication information and set a medication alarm to provide a medication alarm at the set time.

The kiosk 60 may print a medication instruction (S665). Specifically, the kiosk 60 may print a medication instruction including medication information and provide it to the user. The kiosk 60 may transmit a medicine delivery completion response signal to the pharmacy system 40 (S670).

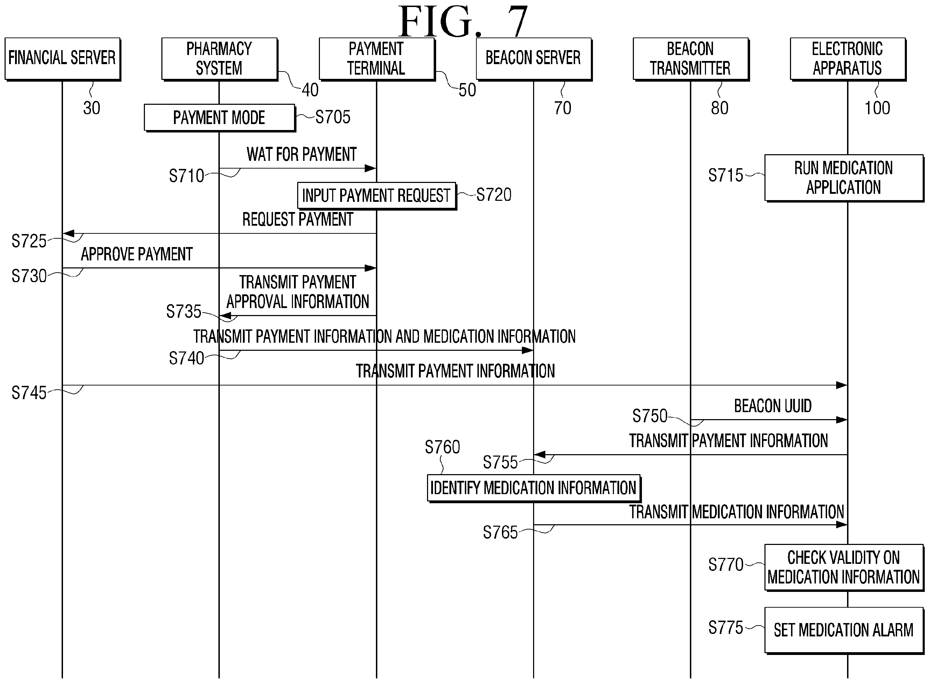

FIG. 7 is a view illustrating an embodiment of providing medication information using a beacon server according to an embodiment of the disclosure.

The pharmacy system 40 may enter a payment mode (S705). Specifically, when acquiring medication information of a patient from an external server or acquiring medication information from a prescription, the pharmacy system 40 may enter a payment mode for paying for medicine

While maintaining the payment mode, the pharmacy system 40 may request the payment terminal 50 to wait for payment (S710). In this case, the pharmacy system 40 may wait to transmit the medication information through the beacon server 70.

The electronic apparatus 100 may run the medication application (S715). In this case, the electronic apparatus 100 may run the medication application according to a user input, but this is only an embodiment, and may automatically run the medication application based on the location information of the electronic apparatus 100.

The payment terminal 50 may receive a payment request (S720). In this case, when a general credit card is detected, the payment terminal 50 may receive a payment request. However, this is only an embodiment, and the payment terminal 50 may receive a payment request by input of a pharmacist or a payment token received from the electronic apparatus 100.

The payment terminal 50 may request payment to the financial server 30 (S725).

When payment is approved based on the information included in the payment token, the financial server 30 may transmit a payment approval signal to the payment terminal 50 (S730). In this case, the payment approval signal may include information on payment details. The payment terminal 50 may transmit a payment approval signal to the pharmacy system 40 (S735). Meanwhile, the financial server 30 may transmit payment information including payment details to the electronic apparatus 100 (S745). In this case, the financial server 30 may transmit payment information including payment details as a text message.

The pharmacy system 40 may transmit the medication information to the beacon server 70 (S740). In this case, the pharmacy system 40 may transmit the medication information to the beacon server 70 after payment is completed, but this is only an embodiment, and the pharmacy system 40 may transmit the medication information to the beacon server 70 before payment is completed.

The beacon transmitter 80 may transmit a beacon universally unique identifier (UUID) (S750). In this case, the beacon transmitter 80 may transmit the beacon UUID at a predetermined period (e.g., once per second), and the electronic apparatus 100 may receive the beacon UUID received from the beacon transmitter 80.

When the beacon UUID is received, the electronic apparatus 100 may transmit payment information to the beacon server 70 in response to the beacon UUID (S755). In this case, the payment information may include information on payment date and time and amount of payment.

The beacon server 70 checks medication information corresponding to the payment information based on the payment information received from the electronic apparatus 100 (S760). In other words, the beacon server 70 may check medication information corresponding to the payment details transmitted by the electronic apparatus 100 among medication information transmitted by the pharmacy system 40.

The beacon server 70 may transmit medication information to the electronic apparatus 100 (S765).

As described with reference to FIG. 4, the electronic apparatus 100 may check the validity of the medication information (S770) and set a medication alarm (S775).

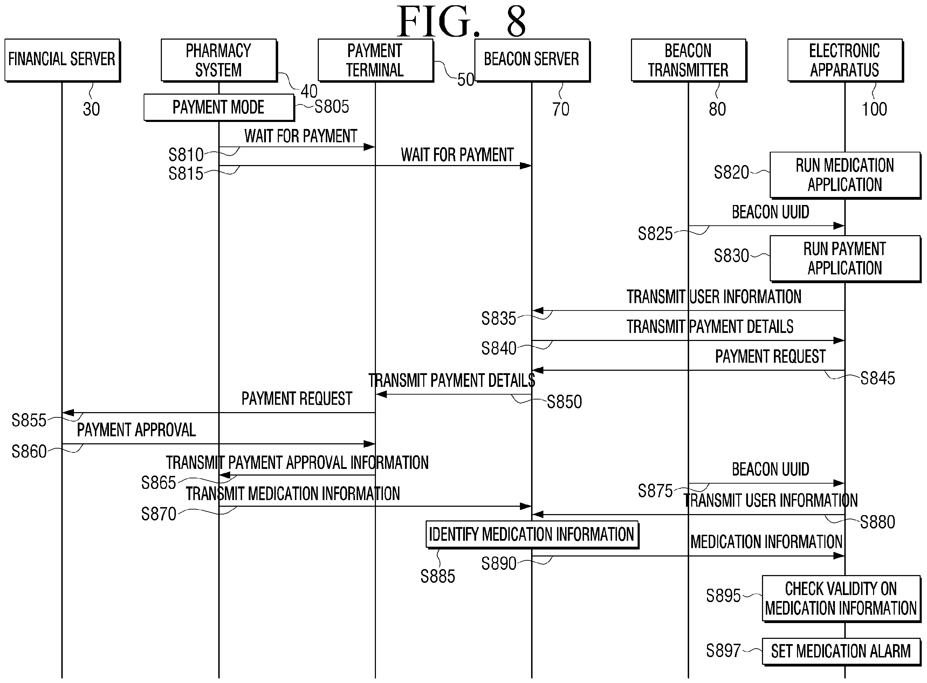

FIG. 8 is a view illustrating another embodiment of providing medication information using a beacon server according to an embodiment of the disclosure.

The pharmacy system 40 may enter a payment mode (S805). Specifically, when acquiring medication information of a patient from an external server or acquiring medication information from a prescription, the pharmacy system 40 may enter a payment mode for payment of the medication.

While maintaining the payment mode, the pharmacy system 40 may request the payment terminal 50 to wait for payment (S810). In this case, the pharmacy system 40 may wait to transmit the medication information.

In addition, the pharmacy system 40 may request the beacon server 70 to wait for payment (S815). In this case, the beacon server may wait to perform payment for medicine. In this case, the pharmacy system 40 may transmit information on a user ID of the application and payment information for the medicine to the beacon server 70 together.

Meanwhile, the electronic apparatus 100 may run the medication application (S820). In this case, the medication application may be run by a user input, but this is only an embodiment and may be run based on the location of the electronic apparatus 100.

The beacon transmitter 80 may transmit the beacon UUID (S825). In this case, information on the beacon server may be stored in the beacon UUID.

The electronic apparatus 100 may execute a payment application based on the beacon UUID (S830). In this case, the payment application and the medication application may be logged in with the same user ID.

In addition, the electronic apparatus 100 may transmit user information to the beacon server 70 (S835). In this case, the user information may include information on the user ID of the application.

The beacon server 70 may transmit payment details to the electronic apparatus 100 based on user information (S840).

The electronic apparatus 100 may transmit a payment request to the beacon server 70 (S845). In this case, the payment request may include payment details and information for initiating payment (e.g., user ID).

The beacon server 70 may transmit payment details to the payment terminal 50 (S850).

The payment terminal 50 may request payment to the financial server 30 (S855). In this case, the payment terminal 50 may transmit information on the payment details to the financial server 30 together.

The financial server 30 may transmit a payment approval signal to the payment terminal 50 (S860). In this case, the payment approval signal may include information on payment details. The payment terminal 50 may transmit payment approval information to the pharmacy system 40 (S865). The pharmacy system 40 may transmit the medication information to the beacon server 70 (S870). In this case, the pharmacy system 40 may transmit the medication information to the beacon server 70 after payment is completed, but this is only an embodiment, and the medication information may be transmitted to the beacon server 70 before the payment is completed.

The beacon transmitter 80 may transmit a beacon universally unique identifier (UUID) (S875). In this case, the beacon transmitter 80 may transmit the beacon UUID at a predetermined period (e.g., once per second), and the electronic apparatus 100 may receive the beacon UUID received from the beacon transmitter 80.

When the beacon UUID is received, the electronic apparatus 100 may transmit user information to the beacon server 70 in response to the beacon UUID (S880). In this case, the user information may include a user ID.

The beacon server 70 identifies medication information corresponding to the payment information based on user information transmitted by the electronic apparatus 100 (S885). In other words, the beacon server 70 may identify medication information corresponding to the user information transmitted by the electronic apparatus 100 among the medication information transmitted by the pharmacy system 40.

The beacon server 70 may transmit the medication information to the electronic apparatus 100 (S890).

As described with reference to FIG. 4, the electronic apparatus 100 may check the validity of the medication information (S895) and set the medication alarm (S897).

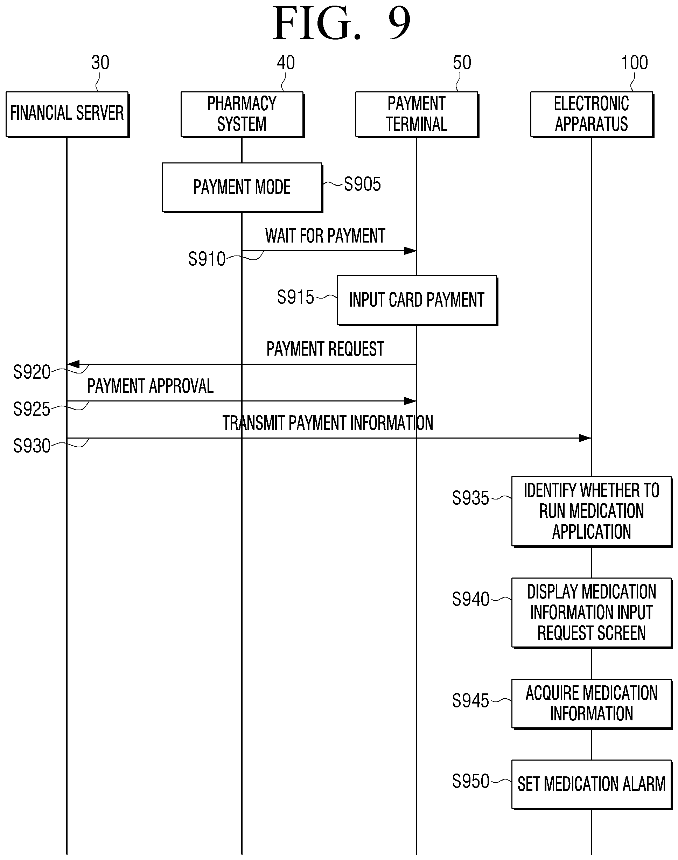

FIG. 9 is a view illustrating an embodiment that medication information is directly input by the user.

The pharmacy system 40 may enter a payment mode (S905). Specifically, when acquiring medication information of a patient from an external server or acquiring medication information from a prescription, the pharmacy system 40 may enter a payment mode for payment of medication.

While maintaining the payment mode, the pharmacy system 40 may request the payment terminal 50 to wait for payment (S910).

The payment terminal 50 may receive a payment request (S915). In this case, when a general credit card is detected, the payment terminal 50 may receive a payment request. However, this is only an example, and the payment terminal 50 may receive a payment request by input of a pharmacist or a payment token received from the electronic apparatus 100.

The payment terminal 50 may request payment to the financial server 30 (S920).

When payment is approved based on information included in the payment token, the financial server 30 may transmit a payment approval signal to the payment terminal 50 (S925). In this case, the payment approval signal may include information on payment details.

The financial server 30 may transmit payment information to the electronic apparatus 100 (S930). In this case, the financial server 30 may transmit the payment information including payment details through short message service (SMS) or multimedia message service (MMS).

The electronic apparatus 100 may identify whether to run the medication application (S935). Specifically, the electronic apparatus 100 may identify whether to run the medication application based on payment information received from the financial server 30 and location information of the electronic apparatus. For example, when texts "pharmacy" and "hospital" are included in the payment information, and payment amount is greater than or equal to the threshold value, the electronic apparatus 100 may run the medication application. In addition, if it is identified that a current location of the electronic apparatus 100 is a hospital or a pharmacy based on GPS and connected network information, the electronic apparatus 100 may run the medication application. Alternatively, if it is identified the location information of the electronic apparatus is identified as "hospital" or "pharmacy" after purchasing medicine, the electronic apparatus may run the medication application at the time of going home, and provide a UI for inputting medication information to the user.

When the medication application is run, the electronic apparatus 100 may display a medication information input request screen (S940). In this case, the medication information input request screen may include a UI element for inputting a type of medicine, a medication time (before or after a meal), a medication method, or the like. Alternatively, the medication information input request screen may include a guide message for inducing photographing of a QR code, a barcode of a prescription including medication information, or the like.

The electronic apparatus 100 may acquire medication information through the medication information input request screen (S945). Specifically, the electronic apparatus 100 may acquire medication information by a user input, and when the QR code or barcode of a prescription is photographed, the electronic apparatus 100 may acquire medication information based on the pre-stored medication information.

The electronic apparatus 100 may set a medication alarm based on the acquired medication information (S950).

As in the embodiment described above, the electronic apparatus 100 may acquire medication information through various methods, and may provide a medication alarm based on the acquired medication information.

In particular, the electronic apparatus 100 may provide a medication alarm according to the user's context. Specifically, the electronic apparatus 100 may provide the medication alarm according to the type of apparatus the user uses.

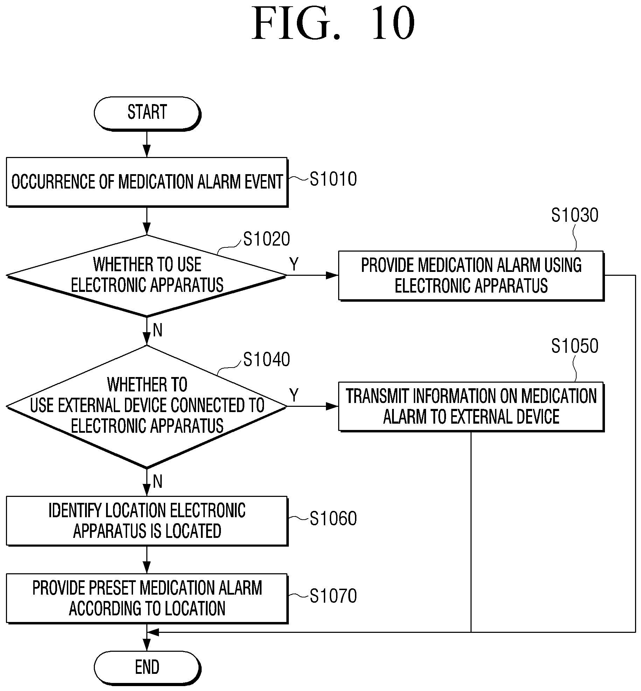

FIG. 10 is a view illustrating an embodiment of providing a medication alarm according to an apparatus used by a user according to an embodiment of the disclosure.

The electronic apparatus 100 may detect an occurrence of a medication alarm event (S1010). Specifically, when an alarm time determined based on the medication information is reached, the electronic apparatus 100 may detect the occurrence of the medication alarm event.

The electronic apparatus 100 may identify whether the user is carrying the electronic apparatus 100 (S1020). Specifically, the electronic apparatus 100 may identify whether the user uses the electronic apparatus 100, whether a motion of the electronic apparatus 100 is detected, whether biometric information is detected through the sensor 160 included in the electronic apparatus 100, or the like.

If it is identified that the user carries the electronic apparatus 100 (S1020-Y), the electronic apparatus 100 may provide a medication alarm using the electronic apparatus 100 (S1030). Specifically, the electronic apparatus 100 may provide a message alarming medication through the display 120 and may provide the alarming message through the speaker 130. Alternatively, the electronic apparatus 100 may provide a vibration feedback for the medication alarm using a vibrating unit.

If it is identified that the user does not carry the electronic apparatus 100 (S1020-N), the electronic apparatus 100 may identify whether to use an external device connected to the electronic apparatus 100 (S1040). Specifically, the electronic apparatus 100 may identify whether to use the external device by identifying a location of the user photographed by a camera of the external device connected to the electronic apparatus 100 and whether a user command is input to the external device. In particular, when the user does not carry the electronic apparatus 100, the electronic apparatus 100 may identify whether to use the external device by transmitting a query to the external device to inquire whether the user uses the external device.

When it is identified that the user is using the external device 100 (S1040-Y), the electronic apparatus 100 may transmit information on the medication alarm to the external device that the user is using (S1050). Accordingly, the external device may provide a medication alarm to the user.

When it is identified that the user does not use the external device 100 (S1040-N), the electronic apparatus 100 may determine a location where the electronic apparatus 100 is located (S1060). In this case, the electronic apparatus 100 may identify the location where the electronic apparatus 100 is currently located based on GPS included in the electronic apparatus 100 or information on a network connected to the electronic apparatus 100.

The electronic apparatus 100 may provide a preset medication alarm according to the identified location (S1070). Specifically, when the electronic apparatus 100 is implemented as a mobile terminal or a wearable device, if it is identified that the electronic apparatus 100 exists outdoors, the electronic apparatus 100 may preferentially provide the medication alarm through the electronic apparatus 100. However, if it is identified that the electronic apparatus 100 is indoors, the electronic apparatus 100 may provide the medication alarm through the preset device (e.g., a robot, an electronic apparatus, etc.).

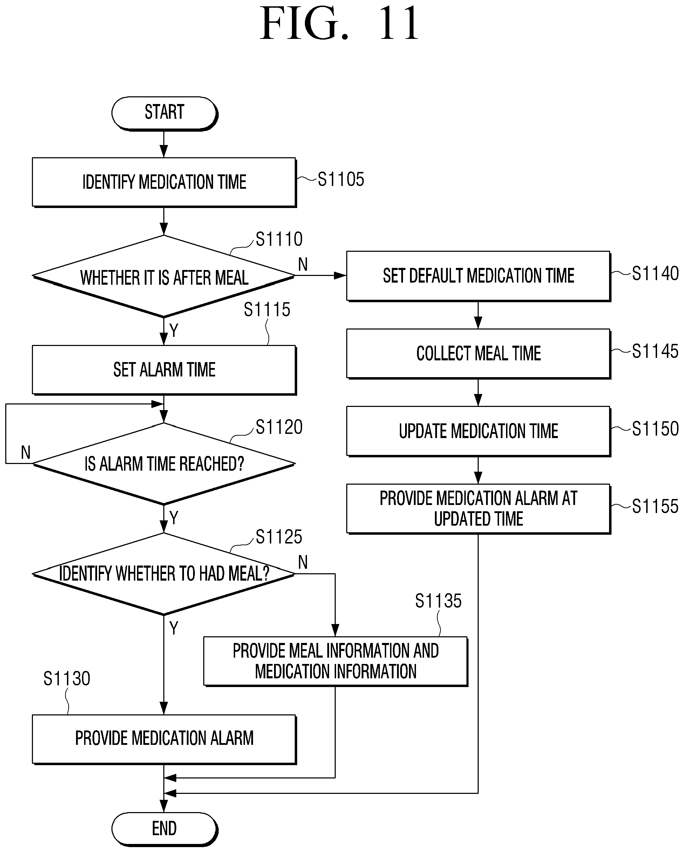

FIG. 11 is a view illustrating an embodiment of providing a medication alarm according to a medication time according to an embodiment of the disclosure.

The electronic apparatus 100 may identify a medication time (S1105). Specifically, the electronic apparatus 100 may identify whether medicine is to be taken before or after a meal based on the time of taking the medicine included in the medication information.

When the medication time is after a meal (S1110-Y), the electronic apparatus 100 may set an alarm time (S1115). Specifically, the electronic apparatus 100 may set a morning time (e.g., 7 to 9 o'clock), a lunch time (e.g., 11 to 13 o'clock), and an evening time (e.g., 18 to 20 o'clock).

In addition, when the alarm time is reached (S1120-Y), the electronic apparatus 100 may identify whether the user had a meal or not (S1125). In this case, the electronic apparatus 100 may identify whether the user had a meal or not based on at least one of image information photographed by the electronic apparatus 100 or an IoT device connected to the electronic apparatus 100, the user's biometric information acquired by the sensor 160 included in the electronic apparatus 100, card payment information received by the electronic apparatus 100. For example, when the electronic apparatus 100 is a smart glass or an external device connected to the electronic apparatus 100 is a robot, the electronic apparatus 100 may analyze an image photographed by the smart glass or the robot to identify whether the user is eating or not. Alternatively, the electronic apparatus 100 may identify whether the user had a meal by identifying whether or not a blood sugar rise above a preset value has continued for a predetermined by using a continuous blood glucose measurement sensor. Alternatively, the electronic apparatus 100 may identify whether to eat by detecting a change in blood sugar through a PPG sensor, an optical sensor, an electromagnetic sensor, an impedance sensor, etc., by detecting whether a body temperature rises through metabolic heat conformation (MHC), and through a measurement patch of tear or sweat. Alternatively, if a "restaurant" or the like is included in the card payment details transmitted to the electronic apparatus 100, the electronic apparatus 100 may identify that the user had a meal.

When the user had a meal (S1125-Y), the electronic apparatus 100 may provide a medication alarm (S1130). When the user did not have a meal (S1125-N), the electronic apparatus 100 may provide meal recommendations and medication information. In particular, when the alarm time has timed out while the user has not eaten, the electronic apparatus 100 may recommend a meal for taking medicine and provide medication information according to the meal. For example, if it is past 13 o'clock without having a meal at lunch time (11 to 13 o'clock), the electronic apparatus 100 may provide a meal recommendation and medication information.

Meanwhile, when the medication time is not after meals (e.g., the medication time is before a meal or not related to whether to eat) (S1110-N), the electronic apparatus 100 may set a default time of medication (S1140). For example, the electronic apparatus 100 may set a general meal time (e.g., 9 am for breakfast, 1 pm for lunch, 6 pm for dinner) as a default medication time.

After the default medication time is set, the electronic apparatus 100 may collect meal time (S1145). In other words, as described above, the electronic apparatus 100 may identify whether the user had a meal, and collect an identified meal time.

The electronic apparatus 100 may update the medication time based on the collected meal time (S1150). For example, if it is identified that the user normally eats breakfast at 8 am, lunch at 12:30 pm, and dinner at 7 pm, the electronic apparatus 100 may update the medication time to 8 am and 12:30 pm, and 7 pm, respectively.

In addition, the electronic apparatus 100 may provide a medication alarm at the updated time (S1155).

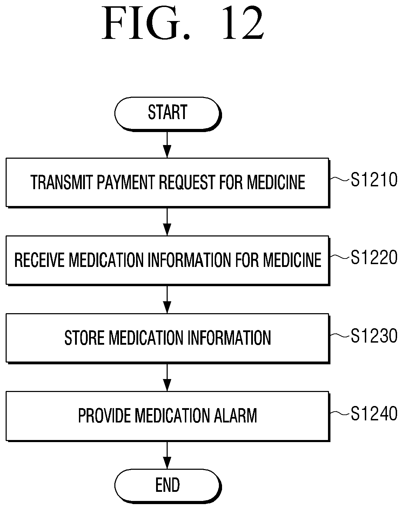

FIG. 12 is a flowchart illustrating a method of controlling an electronic apparatus according to an embodiment of the disclosure.

The electronic apparatus 100 may transmit payment request for medicine (S1210). In this case, the electronic apparatus 100 may request payment to the financial server 30 through the payment terminal 50 using the first communication module 111 (e.g., MST communication module).

The electronic apparatus 100 may receive medication information for the medicine (S1220). Specifically, when the payment is approved from the financial server 30 in response to the payment request, the electronic apparatus 100 may use the second communication module 113 (e.g., NFC communication module) through the payment terminal 50 to receive medication information from the pharmacy system 40.

The electronic apparatus 100 may store the received medication information (S1230). In this case, the electronic apparatus 100 may check validity on the received medication information to store the medication information.

The electronic apparatus 100 may provide a medication alarm based on the medication information (S1240). Specifically, the electronic apparatus 100 may provide a medication alarm at a medication time identified based on a medication time included in medication information.

The various example embodiments described above may be implemented as an S/W program including an instruction stored on machine-readable (e.g., computer-readable) storage media. The machine is an apparatus which is capable of calling a stored instruction from the storage medium and operating according to the called instruction, and may include an electronic apparatus (e.g., an electronic apparatus A) according to the above-described example embodiments. When the instruction is executed by a processor, the processor may perform a function corresponding to the instruction directly or using other components under the control of the processor. The command may include a code generated or executed by a compiler or an interpreter. A machine-readable storage medium may be provided in the form of a non-transitory storage medium. Herein, the term "non-transitory" only denotes that a storage medium does not include a signal but is tangible, and does not distinguish the case where a data is semi-permanently stored in a storage medium from the case where a data is temporarily stored in a storage medium.

According to an embodiment of the disclosure, the methods according to various embodiments described above may be provided as a part of a computer program product. The computer program product may be traded between a seller and a buyer. The computer program product may be distributed in a form of the machine-readable storage media (e.g., compact disc read only memory (CD-ROM) or distributed online through an application store (e.g., PlayStore™). In a case of the online distribution, at least a portion of the computer program product may be at least temporarily stored or provisionally generated on the storage media, such as a manufacturer's server, the application store's server, or a memory in a relay server.

The respective components (e.g., module or program) according to the various example embodiments may include a single entity or a plurality of entities, and some of the corresponding sub-components described above may be omitted, or another sub-component may be further added to the various example embodiments. Alternatively or additionally, some components (e.g., module or program) may be combined to form a single entity which performs the same or similar functions as the corresponding elements before being combined. Operations performed by a module, a program module, or other component, according to various exemplary embodiments, may be sequential, parallel, or both, executed iteratively or heuristically, or at least some operations may be performed in a different order, omitted, or other operations may be added.

## Claims

1. A method of controlling an electronic apparatus comprising:
transmitting a payment request for medicine to an external device;
receive medication information for the medicine from the external device in response to the payment request;
storing the medication information for the medicine; and
providing a medication alarm for the medicine to a user based on the stored medication information.

2. The method of claim 1, wherein the electronic apparatus is configured to transmit information for the payment request to the external device using a magnetic secure transmission (MST) communication model, and receive the medication information for the medicine from the external device using a near field communication (NFC) communication module

3. The method of claim 1, wherein the electronic apparatus is configured to transmit the information on the payment request to the external device using the NFC communication module, and receive the medication information for the medicine from the external device using the NFC communication module.

4. The method of claim 1, comprising:
checking validity on the medication information by comparing user information stored in the electronic apparatus with user information included in the medication information, or comparing the payment information transmitted to the electronic apparatus with the payment information included in the medication information.

5. The method of claim 4, comprising:
setting a medication alarm for the medicine based on the medication information, based on the validity on the medication information being authenticated, and outputting a message guiding that the authentication has been failed based on the validity on the medication information being not authenticated.

6. The method of claim 1, comprising:
identifying an alarm time based on a medication time for the medicine included in the medication information,
wherein the providing includes providing the medication alarm based on the alarm time.

7. The method of claim 6, wherein the providing includes:
identifying whether the alarm time is reached based on the medication time for the medicine being after a meal;
identifying whether the user had a meal based on the alarm time being reached; and
providing the medication alarm after a preset time based on it being identified that the user had a meal.

8. The method of claim 7, wherein the identifying whether the user had a meal includes identifying based on at least one of image information photographed by the electronic apparatus or an IoT device connected to the electronic apparatus, biometric information of the user acquired by a sensor included in the electronic apparatus, and card payment information transmitted to the electronic apparatus.

9. The method of claim 7, comprising providing a message of recommending meals and medication to the user based on it being identified that the user had a meal.

10. The method of claim 6, comprising:
providing the medication alarm at the default medication time included in the alarm time based on the medication time for the medicine being before a meal,
wherein the electronic apparatus is configured to collect meal time of the user of the electronic apparatus and provide the medication alarm at a time updated based on the collected meal time.

11. A computer-readable recording medium for storing computer executable instructions for performing a method of controlling an electronic apparatus, the medium comprising:
transmitting a payment request for medicine to an external device;
storing the medication information for the medicine based on the medication information for the medicine being received from the external device in response to the payment request; and
providing a medication alarm for the medicine to a user based on the stored medication information.

12. The medium of claim 11, wherein information for the payment request is configured to be transmitted to the external device through a MST communication module, and the medication information for the medicine is configured to be received from the external device through a NFC communication module.

13. The medium of claim 11, wherein the information for the payment request is configured to be transmitted by using the NFC communication module, and the medication information for the medicine is configured to be received from the external device by using the NFC communication module.

14. The medium of claim 11, wherein the method of controlling the electronic apparatus includes checking validity on the medication information by comparing user information stored in the electronic apparatus with user information included in the medication information, or comparing the payment information transmitted to the electronic apparatus with the payment information included in the medication information.

15. The medium of claim 14, wherein the method of controlling the electronic apparatus includes setting a medication alarm for the medicine based on the medication information, based on the validity on the medication information being authenticated, and outputting a message guiding that the authentication has been failed based on the validity on the medication information being not authenticated.
